# EUROPÄISCHE PATENTSCHRIFT

(11) **EP 3 675 696 B1**
(45) Veröffentlichungstag und Bekanntmachung des Hinweises auf die Patenterteilung: **07.06.2023**
(21) Anmeldenummer: 18759301.7
(22) Anmeldetag: 23.08.2018
(51) Int. Cl.: A47J 37/12

(54) **FRITTIEREINHEIT**
DEEP-FRYER UNIT
UNITÉ DE FRITURE

(30) Priorität: 29.08.2017 CH 10662017
(43) Veröffentlichungstag der Anmeldung: 08.07.2020
(73) Patentinhaber: FFM Projekt AG, 9500 Wil (CH)
(72) Erfinder: BARANDUN, Urs, 9507 Stettfurt (CH); BINDER, Cyrill, 8046 Zürich (CH)
(74) Vertreter: Troesch Scheidegger Werner AG
(86) Internationale Anmeldenummer: PCT/EP2018/072730
(87) Internationale Veröffentlichungsnummer: WO 2019/042852

(56) Entgegenhaltungen:
- WO-A1-91/09558
- WO-A1-2010/044258
- KR-B1- 101 518 780
- US-A- 5 125 328
- US-B1- 8 584 579

## Beschreibung

### TECHNISCHES GEBIET

Die vorliegende Erfindung betrifft eine Frittiereinheit, insbesondere zum Frittieren von portioniertem Stück- und Schüttgut.

### STAND DER TECHNIK

Aus der US 2016/0278577 ist ein Frittierautomat bekannt, bei welchem ein Korb bei einer Befüll-Station befüllt werden kann, wobei der Korb nach dem Befüllen mittels einer Transporteinheit zu einer von mehreren Frittiereinheiten bewegbar ist und nach dem Frittieren in eine Entleer-Position. Die Frittiereinheiten sind voneinander getrennt und jede Frittiereinheit kann jeweils nur einen Korb zum Frittieren aufnehmen. Jede Frittiereinheit muss separat geheizt werden und die Qualität des Frittieröls ist in jeder Frittiereinheit separat zu gewährleisten. Die grossen Abstände zwischen den Frittiereinheiten und der Befüll- bzw. Entleer-Position führt zu langen Prozesswegen der Transporteinheit, wodurch lange Wartezeiten entstehen. Die langen Verfahrwege müssen bei den Frittierzeiten der einzelnen Frittiereinheiten berücksichtigt werden, da sonst das zu frittierende Gut zu lange frittiert wird und verkohlt. Dies führt zu langen Wartezeiten für die Benutzer eines solchen Frittierautomaten, wenn mehrere Bestellungen hintereinander aufgegeben werden.

Aus der WO 91/09558 ist eine Frittiereinheit bekannt, bei welcher mehrere Körbe gleichzeitig nebeneinander in gemeinsamen oder voneinander getrennten Ölwannen angeordnet werden können. Jeder der Körbe ist mit einer eigenen Transporteinheit von einer Befüll-Position zur Ölwanne und von dieser in eine Entleer-Position verfahrbar. Eine solche Frittiereinheit ist aufwändig bezüglich der Herstellung, der Steuerung und der Wartung.

### BESCHREIBUNG DER ERFINDUNG

Eine Aufgabe der vorliegenden Erfindung besteht darin, eine einfache und kostengünstige Frittiereinheit bereitzustellen, mit welcher die zuvor genannten Nachteile vermieden werden können.

Diese Aufgabe wird durch eine Frittiereinheit mit den Merkmalen des Anspruchs 1 gelöst. Weitere Ausführungsformen der Frittiereinheit, eines Frittierautomaten mit einer erfindungsgemässen Frittiereinheit, sowie ein Verfahren zum Frittieren von Stückgut sind durch die Merkmale von weiteren Ansprüchen definiert.

Eine erfindungsgemässe Frittiereinheit, umfassend
- eine im Wesentlichen oben offene Ölwanne,
- eine Transporteinheit mit welcher ein Korb von einer Befüll-Position in das Innere der Ölwanne bewegbar ist und mit welcher der Korb vom Innern der Ölwanne in eine Entleer-Position bewegbar ist,
dadurch gekennzeichnet, dass die Ölwanne derart ausgebildet ist, dass sie mehrere Körbe gleichzeitig aufnehmen kann und dass die Transporteinheit derart ausgebildet ist, dass die Körbe unabhängig voneinander bewegbar sind.

Somit können mehrere Körbe kurz nacheinander in die gemeinsame Ölwanne eingetaucht werden, was ein zumindest teilweise gleichzeitiges Frittieren erlaubt. Die Ölwanne kann derart bemessen sein, dass zwei, drei, vier, fünf, sechs oder mehr Körbe nebeneinander in ihr angeordnet werden können. In einer gemeinsamen Ölwanne ist das Gesamtvolumen des darin aufnehmbaren Öls grösser, wodurch eine gleichmässigere Öltemperatur und/oder Ölqualität erreichbar ist. Durch die unabhängige Bewegbarkeit der Körbe mit der Transporteinheit kann unterschiedliches Frittiergut und/oder unterschiedliche Mengen an Frittiergut unabhängig voneinander optimal frittiert werden. Die Transporteinheit kann derart ausgebildet sein, dass ein, zwei, drei, vier oder mehr Körbe unabhängig von einander gleichzeitig bewegt, bzw. transportiert werden können. Es ist auch möglich, die Transporteinheit derart zu gestalten, dass Gruppen von Körben unabhängig voneinander bewegbar sind, wobei eine Gruppe von Körben zwei, drei, vier oder mehr Körbe umfassen kann.

In einer Ausführungsform umfasst die Ölwanne einen Boden und an diesen anschliessende und in sich geschlossenen Seitenwände. Im Innern der Ölwanne, beabstandet zum Boden und zu den Seitenwänden ist eine Heizschlange vorgesehen, mit welcher Öl erhitzbar ist, welches sich in der Ölwanne befindet. Die Heizschlange kann vollständig im Innern der Ölwanne angeordnet sein, wobei elektrische Leitungen von der Heizschlange in ein Äusseres der Ölwanne geführt sind. Alternativ kann die Heizschlange vom Äussern der Ölwanne in ihr Inneres führen. Es können mehrere Heizschlangen in unterschiedlichen Bereichen der Ölwanne vorgesehen sein. Die unterschiedlichen Bereiche können zumindest teilweise dichtend durch Trennwände voneinander getrennt sein. Durch den Abstand der Heizschlange vom Boden kann sich unterhalb der Heizschlange eine Zone mit kühlerem Öl bilden. Wenn sich währen dem Frittieren Teile vom Frittiergut trennen, dann sinken diese in der Regel ab. Durch das Absinken gelangen sie in die Zone mit kühlerem Öl, wodurch verhindert werden kann, dass die Teile vollständig verkohlen. Dies wirkt sich positiv auf die Ölqualität aus, da das Verkohlen den Geschmack des Frittieröls und somit des Frittierguts negativ beeinflusst. Die Ölwanne kann isoliert sein, sei es indem sie mit einer Schicht aus Isolationsmaterial umgeben ist und/oder sei es indem sie doppelwandig ausgeführt ist.

Erfindungsgemäss umfasst die Ölwanne Korbaufnahmen, in welchen die Körbe aufgenommen werden können, wobei die Körbe in oberen Korbaufnahmen über dem Öl angeordnet werden können, welches sich in der Ölwanne befindet und wobei die Körbe in unteren Korbaufnahmen in diesem Öl angeordnet werden können. Es können unterschiedliche Korbaufnahmen mit unterschiedlichen Abständen zum Boden der Ölwanne vorgesehen sein, wobei sich die Eintauchtiefe der Körbe durch den Abstand zum Boden einstellen lässt. Die Korbaufnahmen können fix oder bewegbar an der Ölwanne angeordnet sein. Beispielsweise kann eine Korbaufnahme vom Wannenboden wegbewegt werden, wenn die Eintauchtiefe eines darin angeordneten Korbes verringert werden soll. Eine geringere Menge an Frittiergut benötigt eine geringere Eintauchtiefe. Zum Abtropfen des Frittieröls können die Körbe in den oberen Korbaufnahmen angeordnet werden. Die Körbe können auch in den oberen Korbaufnahmen angeordnet werden, wenn sie nicht gebraucht werden. In diesem Fall dienen die oberen Korbaufnahmen als Lagerorte. Beispielsweise können die oberen und/oder unteren Korbaufnahmen Ausnehmungen umfassen, welche sich gegen den Boden der Ölwanne hin verjüngen. Die Körbe werden dadurch in den Aufnahmen zentriert und sicher gehalten.

In einer Ausführungsform umfasst die Transporteinheit eine Vertikalführung, einen Vertikalantrieb und eine mit diesen verbundene Horizontalführung und einen Horizontalantrieb, mit welchen ein Korbhalter horizontal und/oder vertikal bewegbar ist, wobei mit dem Korbhalter ein Korb freigebbar gehalten werden kann. Die Transporteinheit kann auch mehrere horizontale und/oder vertikale Führungen und Antriebe umfassen. Es können auch mehrere Korbhalter vorgesehen sein, welche einzeln oder in Gruppen bewegbar sind. Es ist ebenfalls denkbar, dass eine Vertikalführung und ein Vertikalantrieb mit mehreren Horizontalführungen und Horizontalantrieben kombiniert ist oder dass mehrere Vertikalführungen und mehrere Vertikalantriebe mit einer Horizontalführung und einem Horizontalantrieb kombiniert ist. Der Korbhalter kann schwenkbar oder klemmbar ausgebildet sein.

In einer Ausführungsform ist der Vertikalantrieb und/oder der Horizontalantrieb ein Antrieb, welcher aus der Gruppe auswählbar ist, welche Spindelantrieb, Riemenantrieb, Kettenantrieb, Linearmotor, Linearzylinder und dergleichen umfasst.

In einer Ausführungsform umfasst die Frittiereinheit mindestens einen Korb, wobei der Korb einen Boden und an diesen und aneinander anschliessende Seitenwände umfasst, welche zumindest teilweise öldurchlässig sind. An einem oberen Ende des Korbes ist ein Halter vorgesehen, welcher vom Korbhalter gehalten werden kann. An einem unteren Ende des Korbes ist ein Aufnahmeelement vorgesehen, welches in den Korbaufnahmen aufgenommen werden kann. Der Halter kann in einem oberen Bereich einer Seitenwand angeordnet sein. Das Aufnahmeelement kann in einem unteren Bereich der Seitenwand angeordnet sein, an welcher der Halter angeordnet ist und/oder an einer dieser gegenüberliegenden Seitenwand. Alternativ oder zusätzlich können an den diesen Seitenwänden dazwischenliegenden Seitenwänden mindestens ein Aufnahmeelement angeordnet sein. Es können zwei oder mehr Aufnahmeelemente an einer Seitenwand vorgesehen sein. Es kann auch ein oder mehrere Aufnahmeelemente am Boden des Korbes angeordnet sein.

In einer Ausführungsform ist der Boden des Korbes fix mit den Seitenwänden verbunden oder der Boden umfasst mindestens eine Klappe, welche schwenkbar an einer der Seitenwände angeordnet ist. Es können auch zwei oder mehr schwenkbare Klappen vorgesehen sein.

In einer Ausführungsform umfasst eine erste Seitenwand mindestens eine Klappe, welche schwenkbar an mindestens einer zur ersten Seitenwand benachbarten Seitenwand angeordnet ist oder welche schwenkbar am Boden angeordnet ist. Es können auch zwei oder mehr schwenkbare Klappen vorgesehen sein. An allen Klappen können Rückstell-Elemente, wie beispielsweise Federn vorgesehen sein, um die Klappen in die Schliessstellung zurückzubewegen, wenn sie aus dieser Stellung herausgeschwenkt sind.

In einer Ausführungsform umfasst der Halter zwei im Wesentlichen parallele Stäbe, welche sich vom Korb weg erstrecken. Die Stäbe sind an einer Seitenwand des Korbes angeordnet und erstrecken sich im Wesentlichen senkrecht zu dieser Seitenwand. Jeder der Stäbe kann vom Korbhalter aufgenommen und/oder geklemmt werden. Mit den zwei parallelen Stäben kann verhindert werden, dass sich der Korb während der Bewegung mit der Transporteinheit ungewollt um eine seiner Achsen dreht. Anstelle der beiden Stäbe kann auch ein Halter mit einer Platte oder mit einem Profil vorgesehen sein.

In einer Ausführungsform umfasst die Frittiereinheit eine Ölaufbereitung mit einer Abfuhrleitung, einem an die Abfuhrleitung anschliessenden Filter, eine an den Filter anschliessende Pumpe und eine an die Pumpe anschliessende Rückführleitung, mit welcher Öl aus der Ölwanne abgeführt, gefiltert und zur Ölwanne zurückgeführt werden kann. Beispielsweise kann die Abfuhrleitung im Bereich der kalten Zone angeordnet sein, wodurch das kühlere Frittieröl über den Filter und die Pumpe mittels der Rückführleitung wieder zur Ölwanne zurückgeführt werden kann. Die Rückführleitung kann in einem oberen Bereich der Ölwanne angeordnet sein.

In einer Ausführungsform kann ein oder mehr Sensoren in oder an der Ölwanne angeordnet sein. Die Sensoren können in einem bodennahen Bereich, einem mittleren Bereich oder einem oberen Bereich der Ölwanne angeordnet sein. Es können Temperatursensoren und/oder Ölqualitäts-Sensoren vorgesehen sein. Die Sensoren können alternativ oder zusätzlich auch in der Ölaufbereitung vorgesehen sein.

In einer Ausführungsform umfasst die Frittiereinheit eine Entleer-Einheit mit welcher frittiertes Gut in der Entleer-Position aus dem Korb entleert werden kann.

Beispielsweise umfasst die Entleer-Einheit eine Schwenk-Einheit, mit welcher der Korb um eine Achse schwenkbar ist und/oder die Entleer-Einheit umfasst eine Klapp-Einheit, mit welcher die mindestens eine Klappe des Korbes zum Öffnen und/oder Schliessen betätigbar ist. Mit der Entleer-Einheit kann der Korb beispielsweise um eine seiner horizontalen Hauptachsen geschwenkt werden. Der Schwenkwinkel kann beispielsweise 90 Grad oder 180 Grad betragen. Alternativ oder zusätzlich kann die mindestens eine Klappe des Korbes mit der Entleer-Einheit geöffnet werden, d.h. sie kann um einen Winkel von 90 Grad oder mehr geschwenkt werden. Eine Kombination der Schwenkbewegung des Korbes mit dem Öffnen der Klappe ist ebenfalls möglich. Beispielsweise kann der Boden aufgeklappt werden, ohne dass der Korb gedreht werden muss. Alternativ kann eine Seitenwand des Korbes oder eine in dieser angeordnete Klappe geöffnet werden und der Korb kann um 90 Grad gedreht werden. In einer weiteren Alternative kann der Korb um 180 Grad gedreht werden, ohne dass der Boden oder eine Seitenwand des Korbes geöffnet wird.

In einer Ausführungsform umfasst die Frittiereinheit eine absenkbare Abdeckung, mit welcher die Ölwanne zumindest teilweise abdeckbar ist. Wenn nichts frittiert werden muss, kann die Abdeckung auf die Ölwanne abgesenkt werden, wodurch diese im Wesentlichen oben dicht abgedeckt ist. Dadurch kann verhindert werden, dass das Öl verschmutzt wird und es kann der Wärmeverlust des Öls reduziert werden. Zudem kann verhindert werden, dass Öl und/oder Dampf dem Bereich der Ölwanne entweicht. Alternativ kann die Abdeckung von der Seite über die Ölwanne geschoben werden. Die Abdeckung kann derart ausgebildet sein, dass die Körbe, welche sich in den oberen Korbaufnahmen befinden ebenfalls durch die Abdeckung abgedeckt werden. In diesem Fall umfasst die Abdeckung Seitenwände, welche seitlich über die Körbe ragen und gegen die Ölwanne dichten. Alternativ können die oberen Korbaufnahmen über der Ölwanne angeordnet sein und die Abdeckung kann unter den Körben über die Ölwanne geschoben werden. Die Abdeckung kann isoliert ausgebildet sein, d.h. mit einer Isolationsschicht versehen sein oder doppelwandig ausgebildet sein. Die Abdeckung kann weiter einen Kondensatablauf umfassen, mit welchem Öl und Dampf abgeführt werden kann, welcher an der Oberfläche der Abdeckung kondensiert.

In einer Ausführungsform umfasst die Frittiereinheit eine Lüftung mit einem Lüfter, einem Filter und/oder einem Ölabscheider. Mit der Lüftung kann die Zuluft und/oder Abluft aufbereitet werden. Die Aufbereitung der Zuluft ist aus Gründen der Hygiene und die Aufbereitung der Abluft, um Geruchsemissionen an die Umgebung zu reduzieren oder zu verhindern.

In einer Ausführungsform umfasst die Frittiereinheit ein Gehäuse, welches die zuvor genannten Komponenten der Frittiereinheit zumindest teilweise umgibt. Das Gehäuse kann im Wesentlichen geschlossen ausgebildet sein. Es kann Öffnungen im Bereich der Befüll-Position und im Bereich der Entleer-Position umfassen. Durch die Öffnung bei der Befüll-Position kann Frittiergut der Frittiereinheit zugeführt werden und durch die Öffnung bei der Entleer-Position kann frittiertes Gut aus der Frittiereinheit abgeführt werden. Die Öffnungen können verschliessbar ausgebildet sein. Beispielsweise können sie mit mindestens einem Schieber oder mindestens einer Klappe verschlossen werden. Das Gehäuse kann die zuvor genannte Lüftung umfassen. Die Lüftung kann mehrere Lüfter, Filter und/oder Ölabscheider umfassen und kann im Bereich der Befüll-Position, bzw. der Befüll-Öffnung und/oder im Bereich der Entleer-Position, bzw. der Entleer-Öffnung angeordnet sein.

Ein erfindungsgemässer Frittierautomat umfasst:
- ein Gehäuse,
- ein Dosierapparat, welcher in einem oberen Bereich des Gehäuses angeordnet ist,
- eine Frittiereinheit gemäss einem der vorangehenden Ansprüche, welche unterhalb des Dosierapparates angeordnet ist,
wobei Stückgut, welches den Dosierapparat verlässt, zur Frittiereinheit gelangen kann. Das Stückgut kann durch die Gravitation oder durch ein mechanisches Fördersystem vom Dosierapparat zur Frittiereinheit befördert werden.

In einer Ausführungsform umfasst der Frittierautomat:
- eine Fördereinheit,
- eine Entnahmeeinheit,
- Eine Eingabeeinheit,
- eine Bezahleinheit,
- eine Sicherheitseinheit und
- eine Übermittlungseinheit.

Ein erfindungsgemässes Verfahren zum Frittieren von Stückgut, umfasst die Schritte:
- Bereitstellen einer erfindungsgemässen Frittiereinheit;
- Bewegen des Korbes in die Befüll-Position;
- Befüllen des Korbes mit Stückgut in der Befüll-Position;
- Bewegen des Korbes von der Befüll-Position in das Innere der Ölwanne mit der Transporteinheit;
- Frittieren des Stückguts im Korb im Innern der Ölwanne;
- Bewegen des Korbes vom Innern der Ölwanne in eine Entleer-Position mit der Transporteinheit.

Das frittierte Stückgut kann zum Abtropfen des Frittieröls über der Ölwanne angeordnet werden.

Die erwähnten Ausführungsformen der Frittiereinheit, des Frittierautomaten, sowie des Verfahrens zum Frittieren von Stückgut lassen sich in beliebiger Kombination einsetzen, sofern sie sich nicht widersprechen.

### KURZE BESCHREIBUNG DER FIGUREN

Ausführungsbeispiele der vorliegenden Erfindung werden nachstehend anhand von Figuren noch näher erläutert. Diese dienen lediglich zur Erläuterung und sind nicht einschränkend auszulegen. Es zeigen
Fig. 1 eine perspektivische Darstellung einer erfindungsgemässen Frittiereinheit;
Fig. 2 eine weitere perspektivische Darstellung der Frittiereinheit der Figur 1;
Fig. 3 eine perspektivische Schnittansicht der Frittiereinheit der Figur 1;
Fig. 4 eine perspektivische Ansicht einer Transporteinheit der Frittiereinheit der Figur 1;
Fig. 5 eine perspektivische Darstellung eines Korbes einer ersten Ausführungsform in einer Entleer-Einheit einer ersten Ausführungsform;
Fig. 6 eine perspektivische Darstellung eines Korbes einer zweiten Ausführungsform in einer Entleer-Einheit einer zweiten Ausführungsform; und
Fig. 7 eine perspektivische Darstellung einer erfindungsgemässen Teilschnittansicht eines Frittierautomaten mit einer erfindungsgemässen Frittiereinheit.

### DETAILLIERTE BESCHREIBUNG DER ERFINDUNG

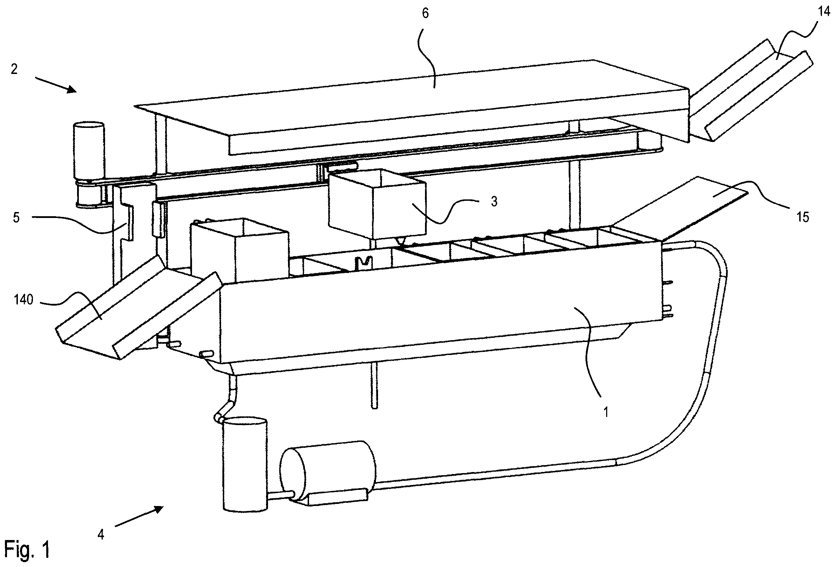

Die Figur 1 zeigt eine schematische Darstellung einer erfindungsgemässen Frittiereinheit. Die Frittiereinheit umfasst eine im Wesentlichen oben offene Ölwanne 1, eine Transporteinheit 2, mit welcher Körbe 3 von einer Befüll-Position in das Innere der Ölwanne 1 bewegbar sind und mit welcher die Körbe 3 vom Innern der Ölwanne 1 in eine Entleer-Position bewegbar sind. Die Ölwanne 1 kann mehrere Körbe 3 gleichzeitig aufnehmen. In der dargestellten Ausführungsform können sechs Körbe 3 gleichzeitig aufgenommen werden. In der Befüll-Position ist eine Einlass-Rutsche 14 vorgesehen, mit welcher das zu frittierende Gut in die Körbe geleitet werden kann. In der Entleer-Position ist eine Auslass-Rutsche 140 vorgesehen, mit welcher das frittierte Gut aus den Körben, beispielsweise zu einer Entnahme-Position geleitet werden kann. Unterhalb der Befüll-Position ist ein Abtropfblech 15 vorgesehen, welches heruntertropfendes Öl, sowie Teile des zu frittierenden Gutes zur Ölwanne leitet, welche aus den Körben fallen. Die Frittiereinheit umfasst weiter eine Ölaufbereitung 4, mit welcher die Qualität des Frittieröls gewährleistet werden kann. Im Bereich der Entleer-Position ist eine Entleer-Einheit 5 vorgesehen, mit welcher das sich in einem Korb 3 befindliche frittierte Gut auf die Auslass-Rutsche 140 bringbar ist. Über der Ölwanne 1 ist eine absenkbare Abdeckung 6 vorgesehen, mit welcher die Ölwanne 1 zumindest teilweise abdeckbar ist, d.h. mit welcher die obere Öffnung der Ölwanne 1 verschliessbar ist.

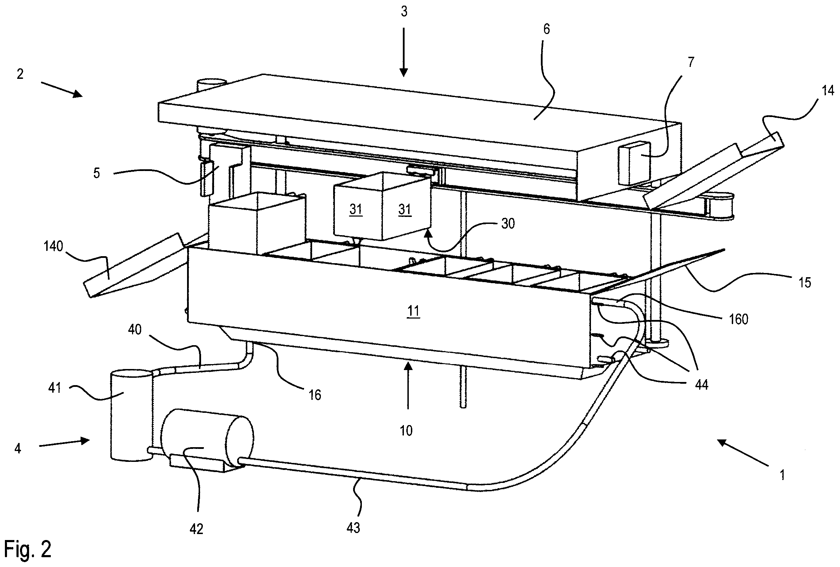

Die Figur 2 zeigt eine weitere perspektivische Darstellung der Frittiereinheit der Figur 1. Die Ölwanne 1 umfasst eine Boden 10 und in sich am Umfang geschlossenen Seitenwände 11, welche sich vom Boden 10 nach oben erstrecken. Jeder Korb 3 umfasst einen Boden 30 und in sich am Umfang geschlossenen Seitenwände 31, welche sich vom Boden 30 nach oben erstrecken. Der Boden 30 und die Seitenwände 31 eines jeden Korbes 3 sind öldurchlässig. Sie können aus einem Metallgeflecht, wie beispielsweise einem Gitter, einem Lochblech oder dergleichen gefertigt sein. Die Ölaufbereitung 4 umfasst eine Abfuhrleitung 40, welche an einem Auslass 16 angeschlossen ist. Der Auslass 16 ist am Boden 10 der Ölwanne 1 angeordnet. Die Abfuhrleitung 40 führt zu einem Ölfilter 41, welcher mit einer Ölpumpe 42 verbunden ist. Von der Pumpe 42 führt eine Rückfuhrleitung zurück zu einem Einlass 160 an der Ölwanne 1 Der Einlass 160 ist im oberen Bereich einer Seitenwand 11 der Ölwanne 1 angeordnet. Das Frittieröl kann von der Ölwanne 1 mit der Ölaufbereitung 4 im Kreislauf gefördert und gefiltert werden. Das Filtrieren kann kontinuierlich oder periodisch erfolgen. Es kann während dem Frittieren erfolgen oder es kann dann erfolgen, wenn die Frittiereinheit nicht in Betrieb ist, d.h. wenn das Öl nicht erhitzt wird. Das Filtrieren kann auch nur dann erfolgen, wenn die Frittiereinheit eine gewisse Zeit ungenutzt war, d.h. wenn sich das Öl unter eine vorgegebene Temperatur abgekühlt hat. An der Ölwanne 1 sind Sensoren 44 vorgesehen. Die dargestellten Sensoren sind an einer Seitenwand 11 der Ölwanne 1 in einem oberen Bereich, in der Mitte und in einem unteren Bereich angeordnet. Die Sensoren können Temperatursensoren oder Ölqualitätssensoren sein. Die Sensoren können auch am Boden 10 der Ölwanne 1 angeordnet sein oder in der Ölaufbereitung 4. An der Abdeckung 6 ist ein Lüftungssystem 7 angeordnet. Das Lüftungssystem 7 umfasst einen Lüfter oder Ventilator und kann zusätzlich noch einen Abscheider für Dampf und/oder Öl umfassen.

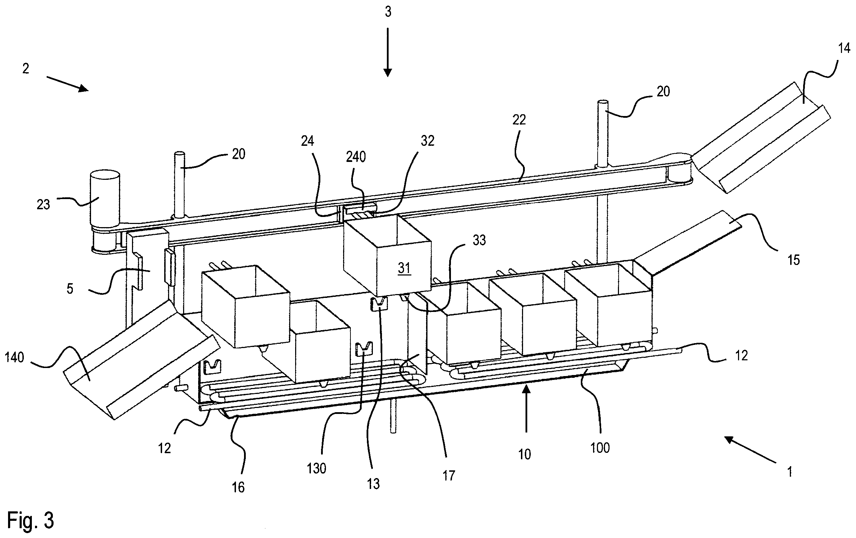

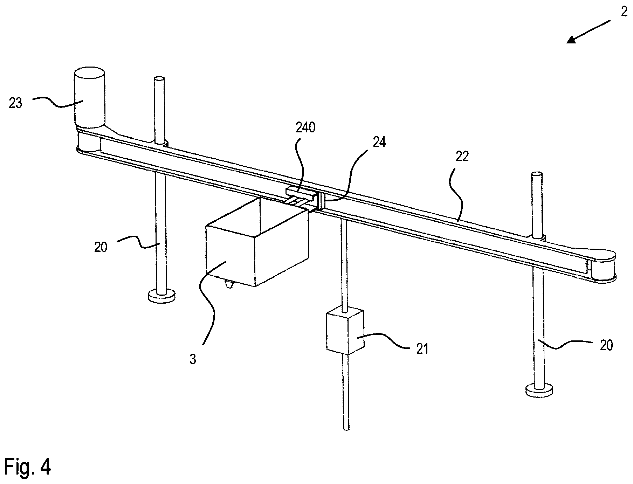

Die Figur 3 zeigt eine perspektivische Schnittansicht der Frittiereinheit der Figur 1. Der Boden 10 der Ölwanne 1 umfasst einen abgesetzten Bereich, welcher eine kalte Zone 100 unterhalb der Heizschlangen 12 bildet. Der abgesetzte Bereich ist beabstandet zu den Seitenwänden 11 im Boden 10 ausgebildet. Der Abstand zu den Seitenwänden 11 bildet eine umlaufende Auflage für die Heizschlangen 12. Die Anschlüsse der Heizschlangen 12 sind seitlich durch die Seitenwände 11 nach aussen geführt. Die dargestellte Ölwanne 1 ist durch eine in der Mitte angeordnete Trennwand 17 in zwei Teile unterteilt. Die Trennwand 17 erstreckt sich über die gesamte Höhe der Ölwanne 1 und unterteilt diese in zwei voneinander getrennte Wannen. Jede der Wannen ist mit einem separaten Anschluss mit der Ölaufbereitung 4 verbunden (dargestellt ist nur eine Verbindungsleitung einer der Wannen). Alternativ kann sich die Trennwand nur über die Höhe der Seitenwände 11 erstrecken, jedoch nicht bis in den abgesetzten Bereich der Ölwanne 1, welcher die kalte Zone 100 bildet, sodass das Frittieröl in der gesamten kalten Zone 100 ungehindert zirkulieren kann. Bei einer geringen Auslastung der Frittiereinheit kann beispielsweise nur ein Teil der Wanne in Betrieb sein. Somit kann der Energieverbrauch verringert werden. Für die Aufbereitung, d.h. die Reinigung und/oder das Auswechseln des Frittieröls kann ein Wannenteil abgeschaltet werden. Wenn sich das Öl genügend abgekühlt hat, kann die Aufbereitung/Auswechslung vorgenommen werden. Anschliessend kann der andere Wannenteil abgeschaltet werden. Auf der Innenseite der Ölwanne 1 sind an einer vorderen Seitenwand (nicht dargestellt) und an einer hinteren Seitenwand Korbaufnahmen 13,130 vorgesehen, in welche die Körbe 3 mit entsprechenden Aufnahmeelementen 33 angeordnet werden können. Es sind obere Korbaufnahmen 13 und untere Korbaufnahmen 130 vorgesehen, wobei sich die Körbe 3 in der Ölwanne 1 befinden, wenn sie in der unteren Korbaufnahme 130 angeordnet sind und sich über der Ölwanne 1, bzw. über dem Frittieröl befinden, wenn sie in der oberen Korbaufnahme 13 angeordnet sind. Zum Abtropfen des Frittieröls können die Körbe 3 in den oberen Korbaufnahmen 13 angeordnet werden. Die oberen und unteren Korbaufnahmen sind seitlich versetzt zueinander angeordnet, d.h. sie sind nicht übereinander angeordnet. Jede Korbaufnahme umfasst eine keilförmige Ausnehmung, welche sich von oben nach unten verjüngt. Die Aufnahmeelemente 33 sind mittig am Boden 30 des jeweiligen Korbes 3 angeordnet. Sie stehen über eine vordere, bzw. hintere Seitenwand 31 des Korbes 3. Im oberen Bereich der hinteren Seitenwand ist am Korb 3 ein Halter 32 vorgesehen, welcher von einem Korbhalter 240 aufgenommen werden kann. Der Korbhalter 240 ist Bestandteil der Transporteinheit 2, welche in der Figur 4 freigestellt dargestellt ist. Die Transporteinheit 2 umfasst zwei Vertikalführungen 20, welche beabstandet zueinander angeordnet sind, einen Vertikalantrieb 21 in der Form eines Spindelantriebs, mit welchem eine Horizontalführung 22, welche mit den Vertikalführungen 20 geführt ist, vertikal verschiebbar ist. Weiter umfasst die Transporteinheit 2 einen Schlitten 24, welcher mit der Horizontalführung 22 geführt ist und welcher mit einem Horizontalantrieb 23, in der Form eines Riemenantriebs horizontal bewegbar ist. Der Korbhalter 240 ist am Schlitten 24 angeordnet, der in der Vertikalen und in der Horizontalen bewegbar ist.

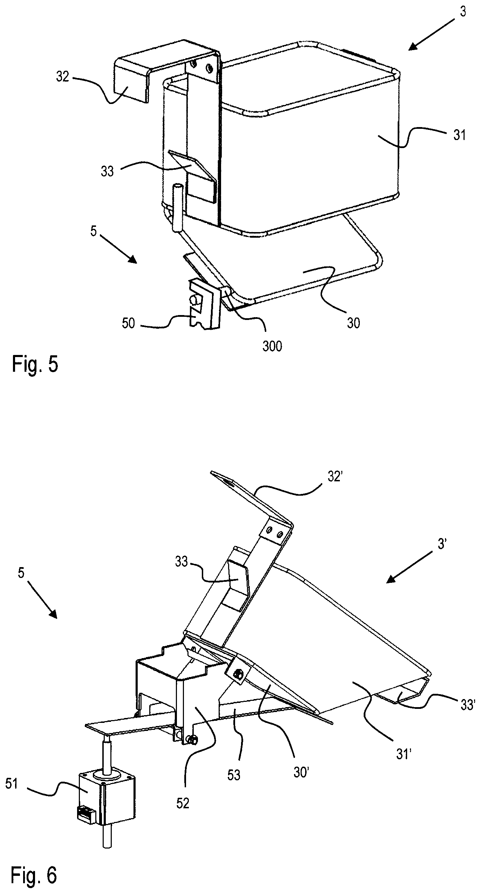

Die Figur 5 zeigt eine perspektivische Darstellung eines Korbes 3 einer ersten Ausführungsform in einer Entleer-Einheit 5 einer ersten Ausführungsform. Der Korb 3 umfasst einen nach unten aufschwenkbaren Boden 30 und geschlossen umlaufende Seitenwände 31. Der Boden 30 hat einen geschlossen umlaufenden Rahmen, in welchem ein Gitter aufgespannt ist. Der Boden 30 wird durch eine Feder in der geschlossenen Position gehalten. Alternativ kann der Boden 30 mit einem Magneten geschlossen gehalten werden, beispielsweise mit einem hitzebeständigen Permanentmagneten, beispielsweise mit einem Ferritmagneten. Die Seitenwände 31 umfassen einen oberen und einen unteren geschlossen umlaufenden Rahmen, zwischen welchen ein Gitter aufgespannt ist. In dieser Ausführungsform ist der Halter 32 als Blechhaken ausgebildet, dessen erster Schenkel sich von der Unterkante einer der Seitenwände 31 bis über die Oberkante dieser Seitenwand 31 erstreckt. Beabstandet zur Oberkante erstreckt sich das Blech in einem Winkel von 90 Grad, d.h. seitlich weg vom Korb 3 und umfasst an seinem dem Korb 3 abgewandten Ende eine nach unten gerichtete Lasche. Im unteren Bereich des Blechhakens, d.h. im unteren Bereich des Bleches des Halters 32, welches zwischen der Unterkante und der Oberkante des Korbes 3 angeordnet ist, erstreckt sich das Aufnahmeelement 33 schräg seitlich nach oben weg vom Korb 3, beispielsweise unter einem Winkel von 45 Grad. Auf der dem Halter 32 gegenüberliegenden Seite des Korbes 3 ist ebenfalls ein Blech mit einem Aufnahmeelement 33 auf der gleichen Höhe angeordnet (nicht sichtbar in dieser Figur). In der Entleer-Einheit 5 ist ein Öffner 50 vorgesehen, in welcher ein vom Boden 30 abstehender Bolzen 300 eingreifen kann. Nach dem Eingreifen des Bolzens 300 im Öffner 50 kann der Korb 3 mit der Transporteinheit 2 angehoben und allenfalls auch noch seitlich verschoben werden, wodurch der Boden 30 nach unten aufgeschwenkt werden kann, wodurch das sich im Korb befindlichen Frittiergut ausgeleert werden kann. Alternativ kann der Korb 3 in der in der Entleer-Einheit 5 stationär angeordnet werden und ein Aktuator kann den Öffner 50 derart vertikal oder vertikal und horizontal bewegen, dass der Boden 30 geöffnet wird.

Die Figur 6 zeigt eine perspektivische Darstellung eines Korbes 3' einer zweiten Ausführungsform in einer Entleer-Einheit 5' einer zweiten Ausführungsform. Der Korb 3' umfasst einen fest mit den Seitenwänden 31' verbundenen Boden 30', wobei sich drei der Seitenwände 31' im Wesentlichen vom Boden 30' senkrecht nach oben erstrecken und eine Seitenwand 31' sich unter einem Winkel, beispielsweise 45 Grad nach oben und nach aussen erstreckt. An der schrägen Seitenwand ist anschliessend an die Oberkante ein Aufnahmeelement 33' angeordnet. Auf der der schrägen Seitenwand gegenüberliegenden Seitenwand ist ein Halter 32' in der Form eines Winkelblechs angeordnet, wobei sich ein erster Schenkel von der Unterkante des Korbes bis über dessen Oberkante hinaus erstreckt und sich ein an den ersten Schenkel anschliessender zweiter Schenkel im Wesentlichen horizontal vom Korb weg erstreckt. In der Entleer-Einheit 5' ist ein Aktuator 51 vorgesehen, mit welchem ein Schwenkmechanismus 53 betätigbar ist. Die Entleer-Einheit 5' umfasst eine Korbaufnahme 52 in welcher der Korb 3' schwenkbar einsetzbar ist. Der Korb 3' kann mit dem Schwenkmechanismus 53 um eine im Wesentlichen horizontale Achse geschwenkt werden, wodurch das sich im Korb befindlichen Frittiergut ausgeleert werden kann.

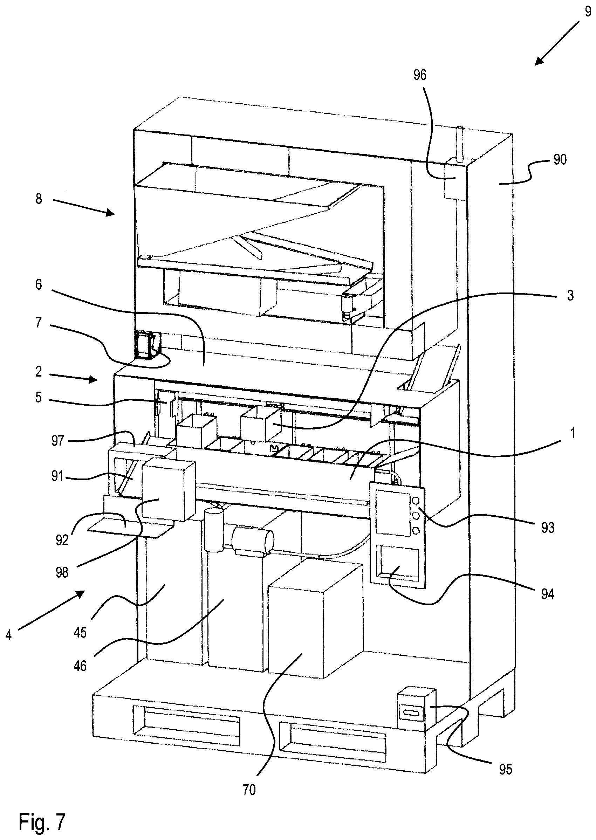

Die Figur 7 zeigt eine perspektivische Teilschnittansicht eines Frittierautomaten 9 mit einer erfindungsgemässen Frittiereinheit. Der Frittierautomat 9 umfasst ein Gehäuse 90 in welchem in einem oberen Bereich ein Dosierapparat angeordnet ist. Die erfindungsgemässe Frittiereinheit ist derart unterhalb des Dosierapparates angeordnet, dass zu frittierendes Gut vom Dosierapparat zur Befüll-Position der Frittiereinheit gelangen kann. Weiter umfasst der Frittierautomat 9 eine an die Frittiereinheit anschliessende Fördereinheit 91, mit welcher das frittierte Gut zu anschliessenden Entnahmeeinheit 92 förderbar ist. Eine Eingabeeinheit 93 und eine Bezahleinheit 94 sind am Gehäuseäussern, in einem für einen Benutzer gut zugänglichen Bereich angeordnet. Im Gehäuseinnern sind eine Sicherheitseinheit 95 und eine Übermittlungseinheit 96 angeordnet. Im Bereich oberhalb der Entnahmeeinheit 91 ist eine Würzeinheit vorgesehen, welche Streuwürze, wie Salz, Pfeffer und andere Gewürze oder Gewürzmischungen umfassen kann oder welche Saucen, wie Ketchup, Mayonnaise, Senf oder andere Saucen umfassen kann, um das Frittiergut damit zu würzen. Neben der Entnahmeeinheit 91 ist eine Schaleneinheit 98 vorgesehen, mit welcher Schalen der Entnahmeeinheit 91 zugeführt werden können, um das Frittiergut darin aufzunehmen. Weiter kann im Frittierautomat im Bereich der Entnahmeeinheit 92 ein Serviettenspender vorgesehen sein (nicht dargestellt), mit welchem Servietten der Entnahmeeinheit 92 zugeführt werden können. Der Serviettenspender kann mit der Schaleneinheit 98 kombiniert ausgebildet sein. Im Frittierautomat 9 sind unterhalb der Frittiereinheit, d.h. unterhalb der Ölwanne 1, ein Frischöl-Behälter 45 und ein Altöl-Behälter 46 angeordnet, welche mit der Ölaufbereitung 4 durch entsprechende Leitungen verbunden sind. Mit entsprechenden Pumpen kann frisches Öl vom Frischöl-Behälter 45 der Ölaufbereitung 4 zugeführt werden und altes Öl kann dem Altöl-Behälter 46 zugeführt werden. In dieser Darstellung ist die Frittiereinheit von einer im Wesentlichen geschlossenen Abdeckung 6, in der Form eines Gehäuses umgeben. Lediglich im Bereich der Zuführung und der Abführung des Frittierguts sind Öffnungen vorgesehen. Das Lüftungssystem 7,70 umfasst einen Lüfter 7, welcher über der Frittiereinheit im Gehäuse 90 des Frittierautomaten 9 angeordnet ist und einen Kondenswasser-Behälter 70, welcher unterhalb der Ölwanne 1 neben den Öl-Behältern 45,46 angeordnet ist. Kondenswasser-Leitungen führen von der Frittiereinheit zum Kondenswasser-Behälter 70.

**BEZUGSZEICHENLISTE**

| | | | |
|---|---|---|---|
| 1 | Ölwanne | 31 | Seitenwand |
| 10 | Boden | 32 | Halter |
| 100 | Kalte Zone | 33 | Aufnahmeelement |
| 11 | Seitenwand | 4 | Ölaufbereitung |
| 12 | Heizelement | 40 | Abfuhrleitung |
| 13 | obere Korbaufnahme | 41 | Filter |
| 130 | untere Korbaufnahme | 42 | Pumpe |
| 14 | Einlass-Rutsche | 43 | Rückfuhrleitung |
| 140 | Auslass-Rutsche | 44 | Sensor |
| 15 | Abtropfblech | 45 | Frischöl-Behälter |
| 16 | Auslass | 46 | Altöl-Behälter |
| 160 | Einlass | 5 | Entleer-Einheit |
| 17 | Trennwand | 50 | Öffner |
| 2 | Transporteinheit | 51 | Aktuator |
| 20 | Vertikalführung | 52 | Korbaufnahme |
| 21 | Vertikalantrieb | 53 | Schwenkmechanismus |
| 22 | Horizontalführung | 6 | Abdeckung |
| 23 | Horizontalantrieb | 7 | Lüftungssystem |
| 24 | Schlitten | 70 | Kondenswasser-Behälter |
| 240 | Korbhalter | | |
| 3 | Korb | 8 | Dosierapparat |
| 30 | Boden | 9 | Frittierautomat |
| | | 90 | Gehäuse |
| 91 | Fördereinheit | 95 | Sicherheitseinheit |
| 92 | Entnahmeeinheit | 96 | Übermittlungseinheit |
| 93 | Eingabeeinheit | 97 | Würzeinheit |
| 94 | Bezahleinheit | 98 | Schaleneinheit |

## Patentansprüche

1. Eine Frittiereinheit, umfassend
- eine im Wesentlichen oben offene Ölwanne (1),
- eine Transporteinheit (2), mit welcher ein Korb (3) von einer Befüll-Position in das Innere der Ölwanne (1) bewegbar ist und mit welcher der Korb (3) vom Innern der Ölwanne (1) in eine Entleer-Position bewegbar ist,
wobei die Ölwanne (1) derart ausgebildet ist, dass sie mehrere Körbe (3) gleichzeitig aufnehmen kann und dass die Transporteinheit (2) derart ausgebildet ist, dass die Körbe (3) unabhängig voneinander bewegbar sind, **dadurch gekennzeichnet, dass** die Ölwanne (1) obere Korbaufnahmen (13) und untere Korbaufnahmen (130) umfasst, in welchen die Körbe (3) aufgenommen werden können, wobei die Körbe (3) in den oberen Korbaufnahmen (13) über dem in der Ölwanne befindlichen Öl angeordnet werden können, welches sich in der Ölwanne (1) befindet und wobei die Körbe (3) in den unteren Korbaufnahmen (130) in diesem Öl angeordnet werden können.

2. Die Frittiereinheit gemäss Anspruch 1, wobei die Ölwanne (1) einen Boden (10) und an diesen anschliessende und in sich geschlossenen Seitenwände (11) umfasst und wobei im Innern der Ölwanne (1), beabstandet zum Boden (10) und zu den Seitenwänden (11) eine Heizschlange (12) vorgesehen ist, mit welcher Öl erhitzbar ist, welches sich in der Ölwanne (1) befindet.

3. Die Frittiereinheit gemäss einem der vorangehenden Ansprüche, wobei die Transporteinheit (2) eine Vertikalführung (20), einen Vertikalantrieb (21) und eine mit diesen verbundene Horizontalführung (22) und einen Horizontalantrieb (23) umfasst, mit welchen ein Korbhalter (240) horizontal und/oder vertikal bewegbar ist, wobei mit dem Korbhalter (240) ein Korb (3) freigebbar gehalten werden kann.

4. Die Frittiereinheit gemäss Anspruch wobei der Vertikalantrieb (20) und/oder der Horizontalantrieb (21) ein Antrieb ist, welcher aus der Gruppe umfassend Spindelantrieb, Riemenantrieb, Kettenantrieb, Linearmotor, Linearzylinder und dergleichen auswählbar ist.

5. Die Frittiereinheit gemäss Anspruch 3 oder 4, umfassend mindestens einen Korb (3), wobei der Korb (3) einen Boden (30) und an diesen und aneinander anschliessende Seitenwände (31) umfasst, welche zumindest teilweise öldurchlässig sind, wobei an einem oberen Ende des Korbes (3) ein Halter (32) vorgesehen ist, welcher vom Korbhalter (240) gehalten werden kann und wobei an einem unteren Ende des Korbes (3) ein Aufnahmeelement (33) vorgesehen ist, welches in den Korbaufnahmen (13,130) aufgenommen werden kann.

6. Die Frittiereinheit gemäss Anspruch 2. f 5, wobei der Boden (30) des Korbes (3) fix mit den Seitenwänden (31) verbunden ist oder wobei der Boden (30) mindestens eine Klappe umfasst, welche schwenkbar an einer der Seitenwände (31) angeordnet ist.

7. Die Frittiereinheit gemäss Anspruch 5, wobei eine erste Seitenwand (31) mindestens eine Klappe umfasst, welche schwenkbar an mindestens einer zur ersten Seitenwand (31) benachbarten Seitenwand (31) angeordnet ist oder welche schwenkbar am Boden (30) angeordnet ist.

8. Die Frittiereinheit gemäss einem der vorangehenden Ansprüche, umfassend eine Ölaufbereitung (4) mit einer Abfuhrleitung (40), einem an die Abfuhrleitung (40) anschliessenden Filter (41), eine an den Filter (41) anschliessende Pumpe (42) und eine an die Pumpe (42) anschliessende Rückführleitung (43), mit welcher Öl aus der Ölwanne (1) abgeführt, gefiltert und zur Ölwanne (1) zurückgeführt werden kann.

9. Die Frittiereinheit gemäss einem der vorangehenden Ansprüche, umfassend eine Entleer-Einheit (5) mit welcher frittiertes Gut in der Entleer-Position aus dem Korb (3) entleert werden kann.

10. Die Frittiereinheit gemäss Anspruch 9, wobei die Entleer-Einheit (5) eine Schwenk-Einheit umfasst, mit welcher der Korb (3) um eine Achse schwenkbar ist oder wobei die Entleer-Einheit (5) eine Klapp-Einheit umfasst, mit welcher die mindestens eine Klappe des Korbes (3) zum Öffnen und/oder Schliessen betätigbar ist.

11. Die Frittiereinheit gemäss einem der vorangehenden Ansprüche, umfassend eine absenkbare Abdeckung (6), mit welcher die Ölwanne (1) zumindest teilweise abdeckbar ist.

12. Die Frittiereinheit gemäss einem der vorangehenden Ansprüche, umfassend eine Lüftung (7) mit einem Lüfter, einem Filter und/oder einem Ölabscheider.

13. Die Frittiereinheit gemäss einem der vorangehenden Ansprüche, umfassend ein Gehäuse, welches die zuvor genannten Komponenten der Frittiereinheit zumindest teilweise umgibt.

14. Ein Frittierautomat (9), umfassend
- ein Gehäuse (90),
- ein Dosierapparat (8), welcher in einem oberen Bereich des Gehäuses (90) angeordnet ist,
- eine Frittiereinheit gemäss einem der vorangehenden Ansprüche, welche unterhalb des Dosierapparates (8) angeordnet ist,
wobei Stückgut, welches den Dosierapparat (8) verlässt, zur Frittiereinheit gelangen kann.

15. Der Frittierautomat (9) gemäss Anspruch 14, umfassend
- eine Fördereinheit (91),
- eine Entnahmeeinheit (92),
- Eine Eingabeeinheit (93),
- eine Bezahleinheit (94),
- eine Sicherheitseinheit (95) und
- eine Übermittlungseinheit (96).

16. Ein Verfahren zum Frittieren von Stückgut, umfassend die Schritte:
- Bereitstellen einer Frittiereinheit gemäss einem der Ansprüche 1 bis 13;
- Bewegen des Korbes (3) in die Befüll-Position;
- Befüllen des Korbes (3) mit Stückgut in der Befüll-Position;
- Bewegen des Korbes (3) von der Befüll-Position in das Innere der Ölwanne (1) mit der Transporteinheit (2);
- Frittieren des Stückguts im Korb (3) im Innern der Ölwanne (1);
- Bewegen des Korbes (3) vom Innern der Ölwanne (1) in eine Entleer-Position mit der Transporteinheit (2).

## Claims

1. A frying unit, comprising
- an oil pan (1) which is essentially open on top,
- a transport unit (2) with which a basket (3) is movable from a filling position into the interior of the oil pan (1) and with which the basket (3) is movable from the interior of the oil pan (1) into an emptying position, wherein the oil pan (1) is configured to accommodate multiple baskets (3) simultaneously and the transport unit (2) is designed in such a way that the baskets (3) are movable independently of one another,
**characterized in that**
the oil pan (1) comprises upper basket receptacles (13) and lower basket receptacles (130) in which the baskets (3) can be accommodated, wherein the baskets (3) can be arranged in upper basket receptacles (13) above the oil, which oil is located in the oil pan (1) and wherein the baskets (3) can be arranged in lower basket receptacles (130) in this oil.

2. The frying unit according to claim 1, wherein the oil pan (1) comprises a bottom (10) and side walls (11) which adjoin thereon and are closed per se and wherein a heating coil (12) is located in the interior of the oil pan (1), spaced from the bottom (10) and the side walls (11) with which oil that is located in the oil pan (1) is heatable.

3. The frying unit according to any of the preceding claims, wherein the transport unit (2) comprises a vertical guide (20), a vertical drive (21), and a horizontal guide (22) connected thereto and a horizontal drive (23), using which a basket holder (240) is movable horizontally and/or vertically, wherein a basket (3) is releasably held using the basket holder (240).

4. The frying unit according to claim 3, wherein the vertical drive (20) and/or the horizontal drive (21) is a drive selectable from the group comprising spindle drives, belt drives, chain drives, linear motors, linear cylinders, and the like.

5. The frying unit according to claim 3 or 4, comprising at least one basket (3), wherein the basket (3) comprises a bottom (30) and side walls (31) adjoining thereon and adjoining each other, which are at least partially oil-permeable, wherein a holder (32) is provided at an upper end of the basket (3), which can be held by the basket holder (240), and wherein a receptacle element (33) is provided at the lower end of the basket (3), which can be accommodated in the basket receptacles (13,130).

6. The frying unit according to claim 5, wherein the bottom (30) of the basket (3) is fixedly connected to the side walls (31) or wherein the bottom (30) comprises at least one flap which is arranged so it is pivotable on one of the side walls (31).

7. The frying unit according to claim 5, wherein a first side wall (31) comprises at least one flap, which is arranged so it is pivotable on at least one side wall (31) adjacent to the first side wall (31) or is arranged so it is pivotable on the bottom (30).

8. The frying unit according to any of the preceding claims, comprising an oil processing unit (4) having a discharge line (40), a filter (41) connected to the discharge line (40), a pump (42) connected to the filter (41) and a return line (43) connected to the pump (42), using which oil can be discharged from the oil pan (1), filtered, and returned to the oil pan (1).

9. The frying unit according to any of the preceding claims, comprising an emptying unit (5), using which fried material can be emptied from the basket (3) in the emptying position.

10. The frying unit according to claim 9, wherein the emptying unit (5) comprises a pivot unit, using which the basket (3) is pivotable around an axis or wherein the emptying unit (5) comprises a flap unit, using which the at least one flap of the basket (3) is actuatable for opening and/or closing.

11. The frying unit according to any of the preceding claims, comprising a cover (6) which can be lowered and with which the oil pan (1) can be at least partially covered.

12. The frying unit according to any of the preceding claims, comprising a ventilation unit (7) having a fan, a filter, and/or an oil separator.

13. The frying unit according to any of the preceding claims, comprising a housing, which at least partially encloses the above-mentioned components of the frying unit.

14. An automatic deep fryer (9), comprising
- a housing (90),
- a metering apparatus (8), which is arranged in an upper region of the housing (90),
- a frying unit according to any of the preceding claims, which is arranged below the metering apparatus (8),
wherein piece goods which leave the metering apparatus (8) can move to the frying unit.

15. The automatic deep fryer (9) according to claim 14, comprising
- a conveyor unit (91),
- a removal unit (92),
- an input unit (93),
- a payment unit (94),
- a security unit (95), and
- a transmission unit (96).

16. A method for frying piece goods, comprising the following steps:
- providing a frying unit according to any of claims 1 to 13;
- moving the basket (3) into the filling position;
- filling the basket (3) with piece goods in the filling position;
- moving the basket (3) from the filling position in the interior of the oil pan (1) using the transport unit (2);
- frying the piece goods in the basket (3) in the interior of the oil pan (1);
- moving the basket (3) from the interior of the oil pan (1) into an emptying position using the transport unit (2).

## Revendications

1. Unité de friture comprenant
- un bac à huile (1) essentiellement ouvert sur le dessus,
- une unité de transport (2) avec laquelle un panier (3) peut être déplacé d'une position de remplissage à l'intérieur du bac à huile (1) et avec laquelle le panier (3) peut être déplacé de l'intérieur du bac à huile (1) à une position de vidange,
dans laquelle le bac à huile (1) est configuré pour accueillir simultanément plusieurs paniers (3) et l'unité de transport (2) est conçue de manière à ce que les paniers (3) puissent être déplacés indépendamment les uns des autres,
**caractérisé en ce que**
le bac à huile (1) comprend des réceptacles de panier supérieurs (13) et des réceptacles de panier inférieurs (130) dans lesquels les paniers (3) peuvent être logés, où les paniers (3) peuvent être disposés dans les réceptacles de panier supérieurs (13) au-dessus de l'huile, laquelle huile est située dans le bac à huile (1), et où les paniers (3) peuvent être disposés dans les réceptacles de panier inférieurs (130) dans cette huile.

2. L'unité de friture selon la revendication 1, dans laquelle le bac à huile (1) comprend un fond (10) et des parois latérales (11) qui se rejoignent et sont fermées en soi, et dans laquelle un serpentin de chauffage (12) est situé à l'intérieur du bac à huile (1), à distance du fond (10) et des parois latérales (11), avec lequel l'huile qui se trouve dans le bac à huile (1) peut être chauffée.

3. L'unité de friture selon l'une des revendications précédentes, dans laquelle l'unité de transport (2) comprend un guide vertical (20), un entraînement vertical (21), un guide horizontal (22) relié à celui-ci et un entraînement horizontal (23), à l'aide duquel un support de panier (240) peut être déplacé horizontalement et/ou verticalement, dans lequel un panier (3) peut être maintenu de manière amovible à l'aide du support de panier (240).

4. L'unité de friture selon la revendication 3, dans laquelle l'entraînement vertical (20) et/ou l'entraînement horizontal (21) est un entraînement pouvant être sélectionné dans le groupe comprenant les entraînements par broche, les entraînements par courroie, les entraînements par chaîne, les moteurs linéaires, les cylindres linéaires, etc.

5. L'unité de friture selon la revendication 3 ou 4, comprenant au moins un panier (3), dans lequel le panier (3) comprend un fond (30) et des parois latérales (31) adjacentes qui sont au moins partiellement perméables à l'huile, dans lequel un support (32) est prévu à une extrémité supérieure du panier (3), qui peut être maintenu par le support de panier (240), et dans lequel un élément de réception (33) est prévu à l'extrémité inférieure du panier (3), qui peut être logé dans les réceptacles de panier (13,130).

6. L'unité de friture selon la revendication 5, dans laquelle le fond (30) du panier (3) est relié de manière fixe aux parois latérales (31) ou dans laquelle le fond (30) comprend au moins un volet qui est disposé de manière à pouvoir pivoter sur l'une des parois latérales (31).

7. L'unité de friture selon la revendication 5, dans laquelle une première paroi latérale (31) comprend au moins un volet, qui est disposé de manière à pouvoir pivoter sur au moins une paroi latérale (31) adjacente à la première paroi latérale (31) ou qui est disposé de manière à pouvoir pivoter sur le fond (30).

8. L'unité de friture selon l'une des revendications précédentes, comprenant une unité de traitement de l'huile (4) ayant une conduite d'évacuation (40), un filtre (41) relié à la conduite d'évacuation (40), une pompe (42) reliée au filtre (41) et une conduite de retour (43) reliée à la pompe (42), à l'aide de laquelle l'huile peut être évacuée du bac à huile (1), filtrée, et renvoyée dans le bac à huile (1) .

9. L'unité de friture selon l'une des revendications précédentes comprend une unité de vidange (5) qui permet de vider la matière frite du panier (3) dans la position de vidange.

10. L'unité de friture selon la revendication 9, dans laquelle l'unité de vidange (5) comprend une unité de pivot, à l'aide de laquelle le panier (3) peut pivoter autour d'un axe, ou dans laquelle l'unité de vidange (5) comprend une unité de volet, à l'aide de laquelle le volet au moins du panier (3) peut être actionné pour s'ouvrir et/ou se fermer.

11. L'unité de friture selon l'une des revendications précédentes, comprenant un couvercle (6) qui peut être abaissé et avec lequel le bac à huile (1) peut être au moins partiellement recouvert.

12. L'unité de friture selon l'une des revendications précédentes, comprenant une unité de ventilation (7) ayant un ventilateur, un filtre et/ou un séparateur d'huile.

13. L'unité de friture selon l'une des revendications précédentes, comprenant un boîtier qui renferme au moins partiellement les composants susmentionnés de l'unité de friture.

14. Friteuse automatique (9), comprenant
- un boîtier (90)
- un appareil de dosage (8), qui est disposé dans une zone supérieure du boîtier (90),
- une unité de friture selon l'une des revendications précédentes, qui est disposée sous l'appareil de dosage (8),
dans lequel les produits en morceaux qui quittent l'appareil de dosage (8) peuvent parvenir à l'unité de friture.

15. Friteuse automatique (9) selon la revendication 14, comprenant
- une unité de transport (91),
- une unité d'enlèvement (92),
- une unité d'entrée (93),
- une unité de paiement (94),
- une unité de sécurité (95), et
- une unité de transmission (96).

16. Méthode de friture de produits en morceaux, comprenant les étapes suivantes :
- fournir une unité de friture selon l'une des revendications 1 à 13 ;
- déplacer le panier (3) dans la position de remplissage ;
- remplir le panier (3) de produits en morceaux dans la position de remplissage ;
- déplacer le panier (3) de la position de remplissage à l'intérieur du bac à huile (1) à l'aide de l'unité de transport (2) ;
- faire frire les produits en morceaux dans le panier (3) à l'intérieur du bac à huile (1) ;
- déplacer le panier (3) de l'intérieur du bac à huile (1) vers une position de vidange à l'aide de l'unité de transport (2).
